# FASCICULE DE BREVET EUROPEEN

(11) **EP 0 946 875 B1**
(45) Date de publication et mention de la délivrance du brevet: **27.10.2004**
(21) Numéro de dépôt: 96942413.4
(22) Date de dépôt: 16.12.1996
(51) Int. Cl.: G01N 33/68

(54) **COMPLEXE HYDROSOLUBLE PROTEINE MEMBRANAIRE-POLYMERE ACRYLIQUE A CARACTERE AMPHIPHILE ET APPLICATION AUX METHODES DE DIAGNOSTIC**
WASSERLÖSLICHER KOMPLEX AUS MEMBRANPROTEINAMPHIPHILE ACRYLPOLYMER UND VERWENDUNG ZUR DIAGNOSE
WATER SOLUBLE ACRYLIC MEMBRANE-POLYMER PROTEIN AMPHIPHILIC COMPLEX AND APPLICATION TO DIAGNOSIS METHODS

(43) Date de publication de la demande: 06.10.1999
(73) Titulaire: CENTRE NATIONAL DE LA RECHERCHE SCIENTIFIQUE, 75016 Paris Cédex (FR)
(72) Inventeur: POPOT, Jean-Luc, F-75013 Paris (FR); TRIBET, Christophe, F-91700 Villiers-sur-Orge (FR); AUDEBERT, Roland, F-95320 Saint Leu la Forêt (FR)
(74) Mandataire: Warcoin, Jacques
(86) Numéro de dépôt international: PCT/FR1996/002009
(87) Numéro de publication internationale: WO 1998/027434

(56) Documents cités:
- EP-A- 0 291 389
- EP-A- 0 363 106
- WO-A-97/04796
- US-A- 5 223 411
- JOURNAL OF IMMUNOLOGICAL METHODS, vol. 93, no. 26, Décembre 1996, NEW YORK US, pages 15047-15050, XP002038264 C. TRIBET ET AL: "Amphipols: polymers that keep membrane proteins soluble in aqueous solutions."

## Description

L'invention concerne de nouveaux complexes hydrosolubles protéines membranaires-polymères vinyliques à caractère amphiphile, un procédé de préparation de ces complexes et l'application de ces complexes aux méthodes de diagnostic ou d'analyse.

Les protéines membranaires intégrales, une classe particulière de protéines, sont insérées *in vivo* dans les membranes biologiques dont elles traversent la double couche lipidique. La surface de ces protéines venant naturellement au contact des membranes (zone transmembranaire) est particulièrement hydrophobe.

Les protéines membranaires assurent des fonctions biologiques essentielles, notamment en ce qui concerne les échanges d'information ou de molécules entre les divers compartiments cellulaires et entre la cellule et son environnement. A ce titre, elles présentent un intérêt majeur dans le domaine médical. Elle représentent, par exemple, des cibles privilégiées pour les molécules médicamenteuses. Elles sont également impliquées dans de nombreuses maladies humaines, dont certaines (par exemple la sclérose en plaque ou la *myasthenia gravis*) ont une composante autoimmune manifestée par la présence dans le sérum d'autoanticorps dirigés contre des protéines membranaires. La manipulation en solution aqueuse des protéines membranaires est le plus souvent un préalable indispensable à leur purification et à leur étude structurale et fonctionnelle. Elle nécessite d'éviter l'agrégation spontanée des domaines hydrophobes et de maintenir autour des zones transmembranaires, un environnement peu polaire.

Les préparations classiques de telles protéines à l'état hydrosoluble contiennent des concentrations micellaires de tensioactifs. Le succès du procédé se fonde sur la grande affinité des régions protéiques transmembranaires pour ces composés amphiphiles et dispersants. Il s'agit néanmoins d'une manipulation plus délicate que dans le cas des protéines solubles, en raison précisément de la présence des tensioactifs.

Ces tensioactifs doivent être additionnés à une concentration supérieure à leur concentration micellaire critique (cmc) à toutes les solutions contenant la protéine étudiée. Outre les éventuels problèmes de coût posés par la consommation de tensioactifs, les expériences sont souvent rendues délicates du fait que les protéines membranaires sont le plus souvent fragiles et sensibles à leur environnement. Par exemple, en présence d'un excès de micelles, elles peuvent se dénaturer, alors qu'un défaut de tensioactif conduit en général à leur précipitation.

Plusieurs brevets, dont on peut citer WO-A-9400557 ; WO-A-9115505; EP-A-363 106 ; DE-A-3 527 139; JP-A-61076500 ; US 5 223 411 ; JP-A-02270856 et JP-A-01168653 décrivent l'extraction, la purification et la manipulation des protéines membranaires en milieu aqueux. Ces protéines sont soit dispersées dans des systèmes micellaires, soit insérées dans des bicouches lipidiques.

Schafmeister et coll. Science, 262, p 734-738 , 1993 ont également décrit la formation de complexes entre protéines membranaires et des polymères amphiphiles peptidiques. Les polymères amphiphiles concernés sont de petits polypeptides nommés peptitergents, structures, rigides (hélices α) dont une face est hydrophobe et l'autre face hydrophile. Les peptitergents maintiennent la solubilité de la bactériorhodopsine. Ils échouent cependant dans le cas d'une porine sans doute parce que leur rigidité limite leurs possibilités d'adaptation face à diverses surfaces hydrophobes. Les auteurs envisagent l'utilisation des peptitergents pour faciliter la cristallisation des protéines membranaires.

On citera également dans le domaine des associations entre polymères synthétiques amphiphiles et protéines globulaires (hydrosolubles) les travaux de F. Petit et coll. Sci., 273, p. 777-781, 1995 sur des polyacrylates modifiés amphiphiles de poids moléculaire compris entre 150 000 et 200 000. L'objet de ces travaux porte sur l'étude des associations protéines/polymères (formation de gels, cinétique et énergétique de la complexation en particulier) et non sur le maintien en solutions disperses de protéines membranaires.

Il existe donc un problème à résoudre en ce qui concerne la manipulation des protéines membranaires dans des solutions aqueuses exemptes de détergent sous la forme de protéines membranaires solubilisées.

L'objet de la présente invention est de résoudre ce problème en général et propose en particulier des complexes nouveaux protéines membranaires-polymères vinyliques à caractère amphiphile qui présentent les avantages suivants :
- réalisation de solutions concentrées de protéines membranaires sous la forme native,
- réalisation de préparations lyophilisées de protéines membranaires sous la forme native,
- faible coût de réalisation.

Les complexes se manipulent en l'absence d'additifs amphiphiles dans le milieu, d'où une réduction des coûts de purification (volumes importants de solutions pour la chromatographie, les dialyses ...).

L'expression "vinylique" englobe dans sa signification générale les polymères acryliques.

L'invention concerne donc un complexe hydrosoluble protéine membranaire-polymère vinylique à caractère amphiphile, caractérisé en ce que ledit polymère vinylique répond à la Formule : 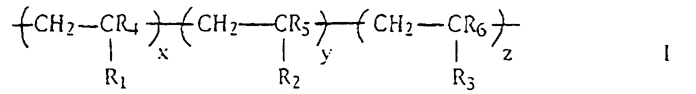 dans laquelle :
R₁ est un groupe :
   - COO^{⊖} M⁺ M⁺ étant un cation alcalin,
   - OOOR₇, R₇ étant un reste sucre ; polyoxyalkylène, notamment polyoxyéthylène, ayant 4 à 10 unités d'oxyde d'alkylène, un radical (CH₂)ₜ-NR₁₀R₁₁, t nombre entier de 1 à 5, R₁₀, R₁₁ identiques ou différents, étant l'atome d'hydrogène, un radical (C₁-C₄) alkyle,
   - N-pyrrolidonyle,
   - phényl sulfonate
   - CONR₈R₉, R₈ et R₉, identiques ou différents étant l'atome d'hydrogène, un reste sucre, polyoxyalkylène, notamment polyoxyéthylène, ayant de 4 à 10 unités d'oxyde d'alkylène, un radical zwittérionique,
R₄, R₅, R₆ identiques ou différents sont l'atome d'hydrogène, ou le radical méthyle,
R₂ est un radical COOR₁₂ ou CONR₁₃R₁₄
   - R₁₂ étant un radical alkyle ou alcényle linéaire ou ramifié de 6 à 12 atomes de carbone,
   - R₁₃, R₁₄ identiques ou différents ont l'une des significations de R₁₂, et de plus l'un des deux peut correspondre à l'atome d'hydrogène,
R₃ est un radical COOR₁₅ ou CONR₁₆R₁₇
   - R₁₅ étant un radical (C₁-C₅) alkyle,
   - R₁₆, R₁₇ ayant l'une des significations de R₁₅ et en plus l'un des deux pouvant correspondre à l'atome d'hydrogène,
x, y, z correspondent aux pourcentages respectifs des motifs,
   - x étant compris entre 20 et 90 %
   - y étant compris entre 10 et 80 %
   - z étant compris entre 0 et 60 %,
La masse molaire moyenne étant comprise entre 500 et 100 000, avantageusement inférieure ou égale à 50 000, de préférence entre 1000 et 50000.

La masse molaire moyenne est donnée en poids.

Les polymères susceptibles d'être utilisés dans le cadre de la présente invention sont donc des polymères amphiphiles qui comportent au moins une chaîne grasse, c'est-à-dire une partie hydrophobe et des motifs hydrophiles, c'est-à-dire une partie hydrophile.

On détaille ci-après à titre indicatif et non limitatif la signification des différents substituants indiqués dans la formule I :
- M⁺ est un cation alcalin lithium, sodium ou potassium,
- R₇ est choisi notamment parmi :
   les restes glucose, fructose, maltose, saccharose et en général les restes mono- ou di-saccharides ; H(̵OCH₂-CH₂)̵_{4,8 ;} (̵CH₂)-N(̵CH₂-CH₃)₂
- R₈, R₉ sont choisis notamment parmi les restes glucosamine, fructosamine, maitosamine, saccharosamine et en général les restes mono- ou di-saccharides aminés ;

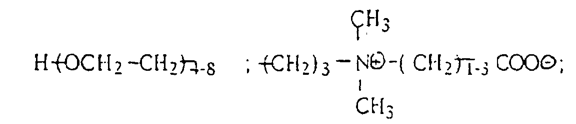

Parmi les radicaux allyle R₁₂ à R₁₄, on cite notamment les radicaux n-hexyle, -n-heptyle, n-octyle, n-nonyle, n-décyle, n-undécyle, n-dodécyle, les radicaux en C₆-C₁₂ possédant un carbone secondaire ou un carbone tertiaire.

Parmi les radicaux alcényle R₁₂ à R₁₄, on cite notamment les radicaux linéaires en C₆-C₁₂ mentionnés ci-dessus possédant une ou deux double liaisons ou les mêmes radicaux possédant un carbone secondaire ou un carbone tertiaire.

Parmi les radicaux alkyle R₁₅ à R₁₇, on cite notamment l'éthyle, l'isopropyle, le n-propyle, le n-butyle, l'isobutyle, le sec-butyle, le t-butyle, le n-pentyle, le sec-pentyle, le t-pentyle, l'isopentyle.

De préférence, l'invention concerne les complexes hydrosolubles protéines membranaires-polymères acryliques à caractère amphiphile, caractérisés en ce que lesdits polymères acryliques répondent à la formule : 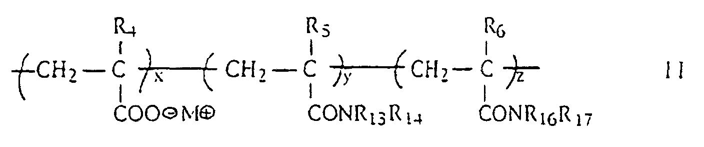 M⁺, R₄, R₅, R₆, R₁₃, R₁₄, R₁₆, R₁₇, x, y, z, ayant la même signification que dans la formule I.

Les polymères acryliques ou vinyliques à caractère amphiphile de formule 1 ou II sont obtenus de manière connue à partir de polymères acryliques ou vinyliques précurseurs, éventuellement disponibles dans le commerce ou synthétisables par polymérisation de monomères vinylique, acrylique ou méthacrylique ou d'un mélange de ces monomères. Dans ce dernier cas, l'on obtient des copolymères qui, par extension, sont inclus dans le terme générique "polymère". Les polymères acryliques à caractère amphiphile de formule II résultent de la réaction de composés R₁₃R₁₄NH et éventuellement R₁₆R₁₇NH avec un polymère acrylique, ce qui conduit à une répartition aléatoire des amides tout au long de la chaîne. Le polymère est préalablement, ou dans une étape ultérieure, mis sous forme salifiée. Un tel mode de synthèse est décrit par exemple dans March , J. (1985) Advanced Organic Chemistry : Reactions, Mechanisms and Structure, pp. 372-374 (Wiley, New York) ; Wang, T.K., lliopoulos, I. & Audebert, R. (1988) Polym. Bull 20, 577-582.

On indique ci-après des variantes préférées de réalisation de l'invention prises ou non en combinaison entre elles ou entre certaines d'entre elles.
- Selon une première variante, R₁₃, R₁₄ correspondent à un atome d'hydrogène ou à un radical alkyle linéaire de 6 à 12 atomes de carbone, R₁₃, R₁₄ ne pouvant être simultanément l'atome d'hydrogène, de préférence R₁₃ ou R₁₄ est un radical n-octyle, l'autre radical R₁₄ ou R₁₃ étant un atome d'hydrogène.
- Selon une seconde variante R₁₆, R₁₇ correspondent à un radical alkyle choisi dans un groupe constitué par les radicaux isopropyle, isobutyle ou à un atome d'hydrogène, R₁₆, R₁₇ ne pouvant simultanément correspondre à l'atome d'hydrogène,
- Selon une troisième variante :
   x est compris entre 30 et 80 %
   y est compris entre 20 et 70 %
   z est compris entre 0 et 50 %,
- Selon une quatrième variante le polymère acrylique à caractère amphiphile est choisi dans le groupe constitué par le polymère dans lequel :
   M⁺ est Na^{⊕} ou K^{⊕}
   R₁₃ est un n-octyle, R₁₄ est H
   x est compris entre 70 et 80 %
   y est compris entre 20 et 30 %
   z est 0 %
   La masse molaire moyenne est comprise entre 2000 et 50 000.
   M⁺ est Na^{⊕} ou K^{⊕}
   R₁₃ est un n-octyle, R₁₄ est H
   R₁₆ est un isopropyle, R₁₇ est H
   x est compris entre 30 et 40 %
   y est compris entre 20 et 30 %
   z est compris entre 30 et 50 %

La masse molaire moyenne est comprise entre 2000 et 50 000.

Le complexe comprend en outre des lipides tels que ceux qui proviennent de la migration de la protéine membranaire lors de la séparation de celle-ci des molécules de détergents.

Le complexe peut comprendre toutes les protéines membranaires d'intérét telles qu'elles ont été définies dans le préambule de la description.

L'invention ne sera donc pas limitée à une catégorie particulière de protéines membranaires. On citera néanmoins particulièrement les protéines membranaires immunogènes, les protéines membranaires de cellules cibles pour les médicaments.

Parmi les protéines membranaires, on cite notamment les protéines mentionnées dans le tableau 4 de Popot et de Vitry (Annu. Rev. Biophys. Biophys. Chem. 1990. 19:369-403) pages 375-378, et leurs homologues eucaryotiques et procaryotiques.

La généralité du mécanisme de maintien en solution aqueuse par les polymères est illustrée par la grande diversité de composition et de structure des protéines les plus étudiées jusqu'à présent : la bactériorhodopsine est un unique polypeptide de MM environ 27.000, associé à une molécule de rétinal. Le centre réactionnel photosynthétique (MM environ 100.000) et le cytochrome b₆f (MM environ 110.000) proviennent de l'association non-covalente de plusieurs polypeptides et d'autres molécules telles que des hèmes, des chlorophylles, des carotènoides... De plus, le b₆f sous sa forme native est un dimère (MM environ 220.000). Les zones transmembranaires de ces trois protéines sont constituées par des faisceaux d'hélices α, tandis que la porine OmpF (un trimère de MM environ 111.000) est une structure en tonneau β. En ce qui concerne la formation des complexes avec les polymères, on n'a relevé aucune différence qualitative entre ces protéines. Un ajustement modeste des concentrations des partenaires s'est bien entendu avéré nécessaire pour optimiser la quantité de polymère utile au maintien en solution de chaque protéine.

L'ajout d'un polymère amphiphile de masse molaire modeste (MM < 50.000 g/mol) même à de faibles concentrations, dans une dispersion de protéines membranaires en milieu micellaire conduit à la formation d'un complexe. Une étape de purification permet d'isoler ce complexe qui comporte la protéine sous forme native, ses cofacteurs ou lipides liés éventuels et plusieurs macromolécules amphiphiles. Le comportement des macromolécules associées aux surfaces hydrophobes des protéines présente les caractéristiques générales de l'adsorption de polymère sur des colloïdes. En particulier, les polymères liés ne se dissocient plus du complexe tant que la solution ne contient pas de molécules concurrentes comme les tensioactifs. La protéine est alors manipulable comme une protéine hydrosoluble, en absence de tensioactifs libres dans le milieu.

La quantité de polymère acrylique à caractère amphiphile par protéine membranaire dépend bien entendu de la taille des polymères et desdites protéines. Certaines protéines sont des dimères, des trimères ou des tétramères et seront donc suceptibles d'accepter des quantités plus ou moins importante de polymère.

Compte tenu de ces précisions, les complexes selon l'invention comprennent en général 1 à 100 molécules de polymère/protéine. avantageusement moins de 10, étant entendu que le nombre de molécules sera fonction de la masse du polymère et de la taille de la protéine.

L'invention concerne encore le complexe, polymère acrylique à caractère amphiphile/protéine membranaire, sous forme lyophilisée.

Elle concerne encore une solution aqueuse hautement concentrée du complexe selon l'invention, avantageusement supérieure à 5 g/l, de préférence comprise entre 10 et 500 g/l. Les protéines membranaires sous ces formes lyophilisées ou hautement concentrées sont à l'état natif et peuvent donc être aisément manipulées et utilisées dans diverses applications.

L'invention concerne également un procédé de préparation des complexes selon l'invention, caractérisé en ce que l'on met en présence une solution de protéines membranaires en milieu détergent sous forme micellaire avec un ou plusieurs polymères vinyliques à caractère amphiphile décrit précédemment, et en ce que l'on abaisse la concentration du détergent à une concentration inférieure à la concentration micellaire critique et on isole le complexe.

L'abaissement de la concentration du détergent peut être réalisé de manière connue : dilution, adsorption des détergents, dialyse, séparation sur tamis moléculaire ou sur gradient par exemple.

La formation de micelles de détergents/protéines membranaires est effectuée de manière connue.

Les complexes de protéines et de polymères peuvent être séparés des tensioactifs présents dans les solutions mères de protéines et du polymère ajouté en excès par diverses méthodes. L'adsorption des molécules amphiphiles, sur des billes Biobeads SM-2 par exemple, permet d'extraire l'essentiel des tensioactifs sans conséquence sur la concentration en polymère. Par dialyse contre une solution tampon de pH, les tensioactifs et les polymères non-liés aux protéines peuvent être extraits en quelques jours du milieu contenant les complexes. Enfin, on peut particulièrement utiliser la sédimentation sur gradient de saccharose. L'analyse des profils de sédimentation indique l'absence d'agrégats parmi les complexes de protéines.

Les quelques molécules résiduelles de tensioactif par protéine sont à comparer aux centaines de molécules de tensioactif liées par protéine en milieu micellaire. Des lipides, qui parfois ont un rôle fonctionnel, peuvent rester associés aux complexes. Dans les complexes solubles obtenus, les polymères apparaissent liés aux protéines dans des proportions indépendantes de la composition initiale du mélange protéine, tensionactif, polymère. Toutefois, la masse de polymère lié par protéine dépend du type de protéine, du pH et de la force ionique.

En général, afin d'assurer une formation efficace des complexes on utilise une solution comportant 0,1 à 10 µM de protéines membranaires et 0,005 % à 1 % de polymère en gramme par gramme de solution. Soit 0,005 g à 1 g de polymère pour 100 g de solution.

L'ajout des polymères doit être effectué avant la dilution de la solution qui se trouve au-dessus de ou à la concentration micellaire critique.

Ce complexe se traduit notamment par le maintien sous forme soluble des protéines après dilution.

La conservation de l'état natif est le plus souvent indispensable pour des applications en biochimie ou dans le domaine médical. On a vérifié la présence dans les complexes purifiés par sédimentation de l'ensemble des sous-unités constitutives de protéines membranaires complexes comme le cytochrome b₆f ou le centre réactionnel. Les spectres d'absorption dans le visible du b₆f ou du centre réactionnel ne subissent pas de modification. Le spectre de la bactériorhodopsine, très sensible à l'environnement de la protéine, présente en présence de polymère, un décalage de quelques nanomètres vers les petites longueurs d'onde, similaire à celui observé en milieu micellaire. On a mesuré l'activité enzymatique de complexes purifiés de b₆f et de polymères. Conservés en solution, les complexes restent actifs durant plusieurs semaines. La vitesse de dégradation est comparable à celle des protéines en milieu micellaire classique.

Les complexes protéines membranaires-polymères acryliques à caractère amphiphile selon l'invention peuvent être utilisés dans diverses applications :
a - simplification de la manipulation des solutions de ces protéines puisque le strict contrôle de la concentration en tensioactifs n'est plus nécessaire.
b - accès à des techniques d'étude des protéines impossibles ou difficiles à mettre en oeuvre en présence de tensioactifs ou de membranes lipidiques (par exemple, la RMN en milieu liquide, cristallogénèse, certaines formes de microscopie électronique...).
c - obtention de solutions de protéines concentrées (> 10 g/l) et fluides.
d - possibilité de lyophiliser les préparations pour les conserver puis de resuspendre les protéines par simple addition d'eau ou de tampon. Ceci ouvre une voie pour la commercialisation des protéines membranaires.
e - de nouveaux systèmes de diagnostic utilisant les protéines membranaires comme récepteurs ou comme antigènes peuvent être envisagés, par exemple pour la recherche d'anticorps circulants, solubles ou portés par des lymphocytes : dans le premier cas, la solubilité de la protéine membranaire en l'absence de détergent facilite la mise au point de protocoles d'immunoprécipitation; dans le second, il est possible de présenter aux cellules l'antigène sous forme soluble sans risquer la lyse cellulaire provoquée par les tensioactifs classiques.
f -les complexes protéines membranaires-polymères amphiphiles présentent potentiellement un grand intérêt en immunologie, pour la présentation de protéines membranaires ou d'autres molécules hydrophobes comme immunogènes aux fins de vaccination ou de production d'anticorps ; des polymères spécialement modifiés pour amplifier la réponse immunitaire sont concevables.
g - de nombreuses protéines membranaires sont des enzymes, dont la complexation par des polymères amphiphiles peut faciliter l'usage soit industriel, soit dans des kits de réactifs.
h - en pharmacologie, la mise à disposition de préparations de protéines membranaires solubles en l'absence de tensioactif est également susceptible de permettre la simplification de nombreux tests, par exemple pour la mesure de l'affinité des récepteurs cellulaires pour des molécules d'intérêt pharmaceutique.

L'invention concerne donc les kits de réactifs comprenant à titre indicatif au moins un complexe selon l'invention, notamment sous forme de solution concentrée ou de préparation lyophilisée.

L'invention concerne également les kits de diagnostic comprenant à titre indicatif au moins un complexe selon l'invention, notamment sous forme de solution concentrée ou de préparation lyophilisée.

L'invention concerne enfin les polymères acryliques à caractère amphiphile nouveaux de formule II décrite ci-dessus, utiles, notamment pour la réalisation de complexes protéines membranaires-polymères acryliques à caractère amphiphile.

Les exemples ci-dessous illustrent l'invention sans toutefois la limiter :

### I - Préparation des polymères acryliques à caractère amphiphile

Les polymères sont préparés par la formation de liaisons amides entre la n-octylamine éventuellement l'isopropylamine et les groupes carboayliques d'acides polyacryliques de bas poids moléculaire :
(masse molaire d'environ 1000 à 20 000 ) selon March, J. et coll. ou Wang, T. K. et coll (op.cit).

Les polymères acryliques à caractère amphiphile obtenus sont des co- ou terpolymères greffés de façon aléatoire le long de la chaine. Les polymères obtenus présentent un caractère amphiphile et ne sont pas insolubles dans l'eau.

Les caractéristiques de certains des polymères obtenus sont réunies dans le tableau ci-dessous.

Le poids moléculaire est mesuré à partir des polyacrylates précurseurs par chromatographie de perméation de gel en utilisant des standards de calibration de polyoxyéthylène de MM de distribution étroite.
MM est la masse molaire apparente en kDa
x, y, z, sont les pourcentages molaires de chaque type d'unités, distribuées de façon aléatoire le long de la chaîne, respectivement non greffée, octylamide, isopropylamide.

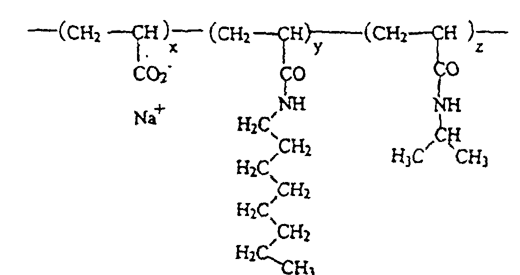

| | MM | x | y | z |
|---|---|---|---|---|
| 1 | 3 | 75 | 25 | 0 |
| 2 | 3 | 35 | 25 | 40 |
| 3 | 8 | 75 | 25 | 0 |
| 4 | 8 | 35 | 25 | 40 |
| 5 | 34 | 75 | 25 | 0 |
| 6 | 34 | 35 | 25 | 40 |

Les valeurs sont indiquées sous réserve des erreurs propres aux mesures.

### II - Préparation des protéines membranaires

- *Bacteriorhodopsine* (BR)
   La membrane de *Halobacterium salinarium* est solubilisée dans 100 mM d'octylthioglucoside (OTG) dans 100 mM de phosphate d'ammonium (PA) à pH 8.0 puis BR est purifiée par centrifugation sur un gradient de saccharose (5-20 % en poids) dans le même tampon contenant 10 mM 0TG (10 h à 54 000 tpm : 250 000 x *g*). La concentration finale était de 0, 1 g/litre dans 100 mM de PA (pH 8.0), environ10 % de saccharose, 10 mM d'OTG.
- Cytochrome b₆f de *chlamydomonas reinhardtii*
   Le complexe Cytochrome b₆f de *Chlamydomonas reinhardtii* est purifié en présence d'Hecameg® et de phosphatidylcholine d'oeuf (PC). La solution finale contient environ 5 µM de complexe b₆f dans 20 mM d'Hecameg®, 0,1 g/l de PCE et 400 mM d'un tampon NaOH/PA et divers inhibiteurs de protéases.
- Centre de réaction photosynthétique de *Rhodobacter sphaeroides* (RC)
   Le centre réactionnel photosynthétique de *Rhodobacter sphaeroides* est en solution à environ 3 g/l dans 20 mM Hecameg® /20 mM tampon NaOH.Tricine, pH 8.0.
- *porine OmpF d'Escherichia coli*
   La *porine OmpF d'Escherichia coli* est en solution à 4 g/l dans 0,2 % en poids d'octylpolyoxyéthylène dans le même tampon que ci-dessus.

### III - Réalisation des complexes

Dans une solution micellaire de protéines membranaires à 0,1 à 5 µM, on ajoute soit 0,05 % en poids de polymères soit 0,005 % puis on dilue dix fois avec du tampon.

Après 15 min d'incubation à 4°C, les solutions sont centrifugées pendant 30 min à 4°C à 210 000 x *g*.

Les concentrations de protéines dans le surnageant sont déterminées à partir de l'absorbance à 564 nm (spectre de différence rédox du cytochrome b₆) ; 546 nm (BR) ; 278 nm (OmpF) ; 802 nm (RC).

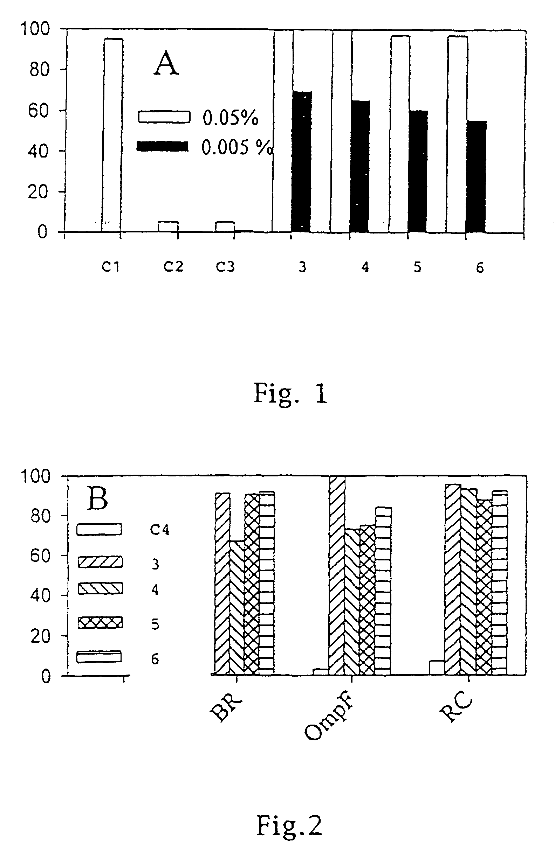

La figure 1 annexée indique le pourcentage en poids de protéines membranaires dans le surnageant avec une comparaison avec :
- C1: une dilution en milieu micellaire,
- C2: une dilution par du tampon sans ajout de polymère,
- C3: une dilution par du tampon après ajout du polyacrylate non greffé.

La protéine membranaire utilisée est le cytochrome b₆f
. en noir 0,005 % de polymère
. en blanc 0,05 % de polymère.

La figure 2 annexée indique le pourcentage en poids de protéines membranaires dans le surnageant (BR, OmpF, RC) en comparaison d'un milieu tampon sans polymère (C4).

Les tests effectués indiquent que ces quatre protéines ainsi stabilisées présentent toutes les caractéristiques des protéines natives.

Après dilution dans 0,25 mM solution LM, les complexes polymères-cytochrome b₆f catalysaient les transferts électroniques du décylplastoquinol à la plastocyanine.

## Revendications

1. Complexe hydrosoluble protéine membranaire-polymère vinylique à caractère amphiphile, **caractérisé en ce que** ledit polymère vinylique répond à la formule : 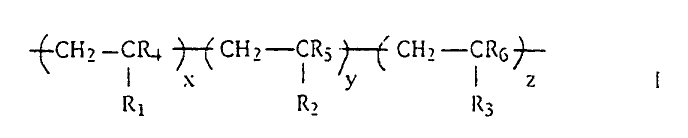 dans laquelle :
R₁ est un groupe :
- COO^{⊖} M⁺ M⁺ étant un cation alcalin,
- COOR₇, R₇ étant un reste sucre ; polyoxyalkylène, notamment polyoxyéthylène, ayant 4 à 10 unités d'oxyde d'alkylène, un radical (CH₂)ₜ -NR₁₀R₁₁, t nombre entier de 1 à 5, R₁₀, R₁₁ identiques ou différents, étant l'atome d'hydrogène, un radical (C₁-C₄) alkyle,
- N-pyrrolidonyle,
- phényl sulfonate
- CONR₈R₉, R₈ et R₉, identiques ou différents étant l'atome d'hydrogène, un reste sucre, polyoxyalkylène, notamment polyoxyéthylène, ayant de 4 à 10 unités d'oxyde d'alkylène, un radical zwittérionique,
R₄, R₅, R₆ identiques ou différents sont l'atome d'hydrogène, ou le radical méthyle,
R₂ est un radical COOR₁₂ ou CONR₁₃R₁₄
- R₁₂ étant un radical alkyle ou alcényle linéaire ou ramifié de 6 à 12 atomes de carbone,
- R₁₃, R₁₄ identiques ou différents ont l'une des significations de R₁₂, et de plus l'un des deux peut correspondre à l'atome d'hydrogène,
R₃ est un radical COOR₁₅ ou CONR₁₆R₁₇
- R₁₅ étant un radical (C₁-C₅) alkyle,
- R₁₆, R₁₇ ayant l'une des significations de R₁₅ et en plus l'un des deux pouvant correspondre à l'atome d'hydrogène,
x, y, z correspondent aux pourcentages respectifs des motifs,
- x étant compris entre 20 et 90 %
- y étant compris entre 10 et 80 %
- z étant compris entre 0 et 60 %
la masse molaire moyenne étant comprise entre 500 et 100000, avantageusement inférieure ou égale à 50 000.

2. Complexe hydrosoluble protéine membranaire-polymère acrylique à caractère amphiphile selon la revendication 1, **caractérisé en ce que** ledit polymère acrylique répond à la formule : 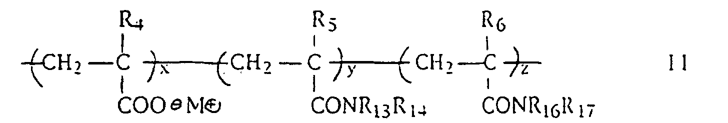 M⁺, R₄, R₅, R₆, R₁₃, R₁₄, R₁₆, R₁₇, x, y, z, ayant la même signification que dans la formule 1 de la revendication 1.

3. Complexe selon l'une des revendications 1 ou 2, **caractérisé en ce que** R₁₃, R₁₄ correspondent à un atome d'hydrogène ou à un radical alkyle linéaire de 6 à 12 atomes de carbone, R₁₃, R₁₄ ne pouvant être simultanément l'atome d'hydrogène.

4. Complexe selon la revendication 3, **caractérisé en ce que** R₁₃ ou R₁₄ est un radical n-octyte, l'autre radical R₁₄ ou R₁₃ étant un atome d'hydrogène.

5. Complexe selon la revendication 1 ou 2, **caractérisé en ce que** R₁₆, R₁₇ correspondent à un radical alkyle choisi dans un groupe constitué par les radicaux isopropyle, isobutyle ou à un atome d'hydrogène, R₁₆, R₁₇ ne pouvant simultanément correspondre à l'atome d'hydrogène.

6. Complexe selon l'une des revendications précédentes, **caractérisé en ce que** :
x est compris entre 30 et 80 %
y est compris entre 20 et 70 %
z est compris entre 0 et 50 %.

7. Complexe selon l'une des revendications 2 à 6, **caractérisé en ce que** le polymère acrylique à caractère amphiphile est choisi dans le groupe constitué par le polymère dans lequel :
M⁺ est Na^{⊕} ou K^{⊕}
R₁₃ est un n-octyle, R₁₄ est H
x est compris entre 70 et 80 %
y est compris entre 20 et 30 %
z est 0 %
la masse molaire moyenne étant comprise entre 2000 et 50 000, ou
M⁺ est Na⊕ou K⊕
R₁₃ est un n-octyle, R₁₄ est H
R₁₆ est un isopropyle, R₁₇ est H
x est compris entre 30 et 40 %
y est compris entre 20 et 30 %
z est compris entre 30 et 50 %
la masse molaire moyenne étant comprise entre 2000 et 50 000.

8. Complexe selon l'une des revendications précédentes, **caractérisé en ce qu'**il comprend en outre des composés lipidiques.

9. Complexe selon l'une des revendications précédentes, **caractérisé en ce qu'**il est sous forme lyophilisée.

10. Solution aqueuse hautement concentrée, de concentration supérieure à 5 g/l, avantageusement entre 10 et 500 g/l d'un complexe selon l'une des revendications 1 à 8.

11. Complexe selon l'une des revendications 1 à 9, **caractérisé en ce que** les protéines membranaires sont choisies dans le groupe constitué par les protéines des eucaryotes et procaryotes, notamment les protéines à activité enzymatique.

12. Procédé de préparation des complexes selon l'une des revendications 1 à 8 ou 11, **caractérisé en ce que** l'on met en présence une solution de protéines membranaires en milieu détergent sous forme micellaire avec un ou plusieurs polymères vinyliques à caractère amphiphile selon l'une des revendications 1 à 7, et **en ce que** l'on abaisse la concentration du détergent à une concentration inférieure à la concentration micellaire critique et on isole le complexe.

13. Kit de réactif comprenant à titre de réactif au moins un complexe selon l'une des revendications 1 à 11.

14. Kit de diagnostic comprenant à titre de réactif immunologique au moins un complexe selon l'une des revendications 1 à 11.

## Patentansprüche

1. Wasserlöslicher Komplex aus Membranprotein-Vinylpolymer mit amphiphilem Charakter, **dadurch gekennzeichnet, dass** das Vinylpolymer die Formel: 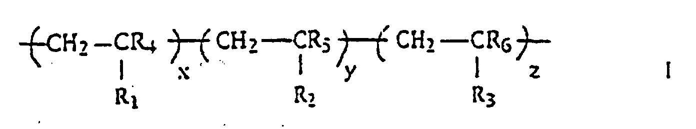 aufweist, in der:
R₁ eine Gruppe:
- COO⁻M⁺, wobei M⁺ ein Alkalikation ist,
- COOR₇, wobei R₇ ein Zuckerrest ; Polyoxyalkylen, insbesondere Polyoxyethylen, mit 4 bis 10 Alkylenoxid-Einheiten; ein Rest (CH₂)ₜ-NR₁₀R₁₁ ist, wobei t eine ganze Zahl von 1 bis 5 ist, R₁₀, R₁₁, identisch oder verschieden, ein Wasserstoffatom, ein (C₁-C₄)-Alkylrest sind,
- N-Pyrrolidonyl,
- Phenylsulfonat,
- CONR₈R₉, wobei R₈ und R₉, identisch oder verschieden, ein Wasserstoffatom, ein Zuckerrest, Polyoxyalkylen, insbesondere Polyoxyethylen, mit 4 bis 10 Alkylenoxid-Einheiten, ein zwitterionischer Rest sind,
darstellt,
R₄, R₅, R₆, identisch oder verschieden, ein Wasserstoffatom oder ein Methylrest sind,
R₂ ein Rest COOR₁₂ oder CONR₁₃R₁₄ ist, wobei
- R₁₂ ein linearer oder verzweigter Alkyl- oder Alkenylrest mit 6 bis 12 Kohlenstoffatomen ist,
- R₁₃, R₁₄, identisch oder verschieden, eine der Bedeutungen von R₁₂ aufweisen und darüber hinaus eines der beiden ein Wasserstoffatom darstellen kann,
R₃ ein Rest COOR₁₅ oder CONR₁₆R₁₇ ist, wobei
- R₁₅ ein (C₁-C₅)-Alkylrest ist,
- R₁₆, R₁₇ eine der Bedeutungen von R₁₅ aufweisen und darüber hinaus eines der beiden ein Wasserstoffatom darstellen kann,
x, y, z den jeweiligen Prozentsätzen der Einheiten entsprechen, wobei
- x 20 bis 90% beträgt,
- y 10 bis 80% beträgt,
- z 0 bis 60% beträgt,
wobei das mittlere Molekulargewicht 500 bis 100000, vorteilhaft weniger als oder gleich 50000 beträgt.

2. Wasserlöslicher Komplex aus Membranprotein-Acrylpolymer mit amphiphilem Charakter nach Anspruch 1, **dadurch gekennzeichnet, dass** das Acrylpolymer der Formel: 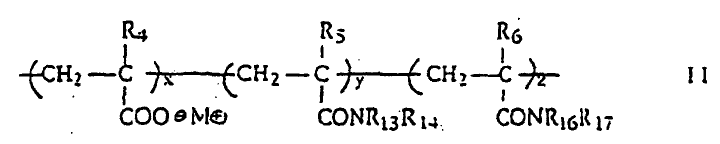 entspricht, wobei M⁺, R₄, R₅, R₆, R₁₃, R₁₄, R₁₆, R₁₇, x, y, z die gleichen Bedeutungen wie in der Formel I von Anspruch 1 aufweisen.

3. Komplex nach einem der Ansprüche 1 oder 2, **dadurch gekennzeichnet, dass** R₁₃, R₁₄ ein Wasserstoffatom oder einen linearen Alkylrest mit 6 bis 12 Kohlenstoffatomen darstellen, wobei R₁₃, R₁₄ nicht gleichzeitig ein Wasserstoffatom sein können.

4. Komplex nach Anspruch 3, **dadurch gekennzeichnet, dass** R₁₃ oder R₁₄ ein n-Octylrest ist, wobei der andere Rest R₁₄ oder R₁₃ ein Wasserstoffatom ist.

5. Komplex nach Anspruch 1 oder 2, **dadurch gekennzeichnet, dass** R₁₆, R₁₇ einen Alkylrest, der aus der Gruppe ausgewählt ist, die aus dem Isopropyl-, Isobutylrest besteht, oder ein Wasserstoffatom darstellen, wobei R₁₆, R₁₇ nicht gleichzeitig ein Wasserstoffatom darstellen können.

6. Komplex nach einem der vorangehenden Ansprüche, **dadurch gekennzeichnet, dass**:
x 30 bis 80% beträgt,
y 20 bis 70% beträgt,
z 0 bis 50% beträgt.

7. Komplex nach einem der Ansprüche 2 bis 6, **dadurch gekennzeichnet, dass** das Acryl-Polymer mit amphiphilem Charakter aus der Gruppe ausgewählt ist, die aus dem Polymer besteht, in welchem:
M⁺ für Na⁺ oder K⁺ steht,
R₁₃ für n-Octyl steht, R₁₄ für H steht,
x 70 bis 80% beträgt,
y 20 bis 30% beträgt,
z 0% beträgt,
wobei das mittlere Molekulargewicht 2000 bis 50000 beträgt,
oder
M⁺ für Na⁺ oder K⁺ steht,
R₁₃ für n-Octyl steht, R₁₄ für H steht,
R₁₆ für Isopropyl steht, R₁₇ für H steht,
x 30 bis 40% beträgt,
y 20 bis 30% beträgt,
z 30 bis 50% beträgt,
wobei das mittlere Molekulargewicht 2000 bis 50000 beträgt.

8. Komplex nach einem der vorangehenden Ansprüche, **dadurch gekennzeichnet, dass** er darüber hinaus lipidische Verbindungen enthält.

9. Komplex nach einem der vorangehenden Ansprüche, **dadurch gekennzeichnet, dass** er in lyophilisierter Form vorliegt.

10. Hochkonzentrierte wässrige Lösung mit einer Konzentration über 5 g/l, vorteilhaft zwischen 10 und 500 g/l, eines Komplexes gemäß einem der Ansprüche 1 bis 8.

11. Komplex nach einem der Ansprüche 1 bis 9, **dadurch gekennzeichnet, dass** die Membranproteine aus der Gruppe ausgewählt sind, die aus Proteinen von Eukaryoten und Prokaryoten, insbesondere Proteinen mit enzymatischer Aktivität, besteht.

12. Verfahren zur Herstellung der Komplexe nach einem der Ansprüche 1 bis 8 oder 11, **dadurch gekennzeichnet, dass** man eine Lösung von Membranproteinen in Detergens-Medium in Mizellenform mit einem oder mehreren Vinylpolymeren mit amphiphilem Charakter gemäß einem der Ansprüche 1 bis 7 in Kontakt bringt und dass man die Konzentration des Detergens auf eine Konzentration unterhalb der kritischen Mizellenkonzentration absenkt und den Komplex isoliert.

13. Reagenzkit, umfassend mindestens einen Komplex nach einem der Ansprüche 1 bis 11 als Reagenz.

14. Diagnostisches Kit, umfassend mindestens einen Komplex nach einem der Ansprüche 1 bis 11 als immunologisches Reagenz.

## Claims

1. Membrane protein-vinyl polymer of amphiphilic nature water-soluble complex, **characterized in that** the said vinyl polymer corresponds to the formula: 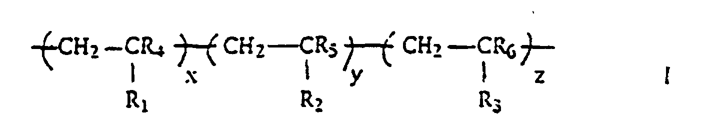 in which:
R₁ is a group:
- COO⁻ M⁺, M⁺ being an alkali metal cation,
- COOR₇, R₇ being a sugar residue; polyoxyalkylene, in particular polyoxyethylene containing 4 to 10 alkylene oxide units, a radical (CH₂)ₜ-NR₁₀R₁₁, t being an integer from 1 to 5, R₁₀ and R₁₁, which may be identical or different, being a hydrogen atom or a (C₁-C₄)alkyl radical,
- N-pyrrolidonyl,
- phenyl sulphonate,
- CONR₈R₉, R₈ and R₉, which may be identical or different, being a hydrogen atom, a sugar residue, polyoxyalkylene, in particular polyoxyethylene containing from 4 to 10 alkylene oxide units, or a zwitterionic radical,
R₄, R₅ and R₆, which may be identical or different, are a hydrogen atom or a methyl radical,
R₂ is a radical COOR₁₂ or CONR₁₃R₁₄
- R₁₂ being a linear or branched alkyl or alkenyl radical of 6 to 12 carbon atoms,
- R₁₃ and R₁₄, which may be identical or different, have one of the meanings of R₁₂, and in addition one of the two can correspond to a hydrogen atom,
R₃ is a radical COOR₁₅ or CONR₁₆R₁₇
- R₁₅ being a (C₁-C₅)alkyl radical,
- R₁₆ and R₁₇ having one of the meanings of R₁₅ and in addition it being possible for one of the two to correspond to a hydrogen atom,
x, y and z correspond to the respective percentages of the units,
- x being between 20 and 90%
- y being between 10 and 80%
- z being between 0 and 60%
the average molar mass being between 500 and 100,000, advantageously less than or equal to 50,000.

2. Membrane protein-acrylic polymer of amphiphilic nature water-soluble complex according to Claim 1, **characterized in that** the said acrylic polymer corresponds to the formula: 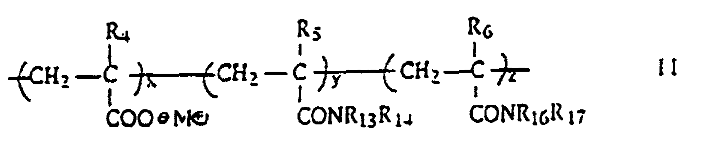 M⁺, R₄, R₅, R₆, R₁₃, R₁₄, R₁₆, R₁₇, x, y and z having the same meaning as in formula I of Claim 1.

3. Complex according to either of Claims 1 and 2, **characterized in that** R₁₃ and R₁₄ correspond to a hydrogen atom or to a linear alkyl radical of 6 to 12 carbon atoms, it not being possible for R₁₃ and R₁₄ simultaneously to be a hydrogen atom.

4. Complex according to Claim 3, **characterized in that** R₁₃ or R₁₄ is an n-octyl radical, the other radical R₁₄ or R₁₃ being a hydrogen atom.

5. Complex according to Claim 1 or 2, **characterized in that** R₁₆ and R₁₇ correspond to an alkyl radical chosen from a group consisting of isopropyl and isobutyl radicals or to a hydrogen atom, it not being possible for R₁₆ and R₁₇ simultaneously to correspond to a hydrogen atom.

6. Complex according to one of the preceding claims, **characterized in that**:
x is between 30 and 80%
y is between 20 and 70%
z is between 0 to 50%.

7. Complex according to one of Claims 2 to 6, **characterized in that** the acrylic polymer of amphiphilic nature is chosen from the group consisting of the polymer in which:
M⁺ is Na⁺ or K⁺
R₁₃ is an n-octyl and R₁₄ is H
x is between 70 and 80%
y is between 20 and 30%
z is 0%
the average molar mass being between 2000 and 50,000, or
M⁺ is Na⁺ or K⁺
R₁₃ is an n-octyl and R₁₄ is H
R₁₆ is an isopropyl and R₁₇ is H
x is between 30 and 40%
y is between 20 and 30%
z is between 30 and 50%
the average molar mass being between 2000 and 50,000.

8. Complex according to one of the preceding claims, **characterized in that** it also comprises lipid compounds.

9. Complex according to one of the preceding claims, **characterized in that** it is in freeze-dried form.

10. Highly concentrated aqueous solution, with a concentration of greater than 5 g/l, advantageously between 10 and 500 g/l, of a complex according to one of Claims 1 to 8.

11. Complex according to one of Claims 1 to 9, **characterized in that** the membrane proteins are chosen from the group consisting of eukaryotic and prokaryotic proteins, in particular proteins with enzymatic activity.

12. Process for preparing the complexes according to one of Claims 1 to 8 or 11, **characterized in that** a solution of membrane proteins in detergent medium in micellar form is placed in contact with one or more vinyl polymers of amphiphilic nature according to one of Claims 1 to 7, and **in that** the concentration of the detergent is lowered to a concentration below the critical micelle concentration and the complex is isolated.

13. Reagent kit comprising, as reagent, at least one complex according to one of Claims 1 to 11.

14. Diagnostic kit comprising, as immunological reagent, at least one complex according to one of Claims 1 to 11.
